# EUROPEAN PATENT APPLICATION

(11) **EP 3 896 150 A1**
(43) Date of publication of application: **20.10.2021**
(21) Application number: 19895178.2
(22) Date of filing: 13.12.2019
(51) Int. Cl.: C12N 1/12, C12N 13/00, C12P 7/64

(54) **METHOD FOR INCREASING THE PRODUCTION OF BIOMASS AND LIPIDS IN CHLORELLA VULGARIS**

(30) Priority: 14.12.2018 MX 2019000561
(71) Applicant: Centro de Investigación Científica y de Educación Superior de Ensenada, Baja California (CICESE), 22860 Ensenada, Baja California (MX)
(72) Inventor: SÁNCHEZ SAAVEDRA, M. del Pilar, Ensenada, Baja California, 22860 (MX)
(74) Representative: Balder IP Law, S.L.
(86) International application number: PCT/MX2019/050030
(87) International publication number: WO 2020/122702

(57) **Abstract**

A method to increase the biomass production and fatty acids in the alga *Chlorella vulgaris* Beyerinck and obtain a lipid rich biomass type C:16 (palmitic), C18:1n-9 (oleic), C18:2n-6t (linoleidic) and C18:3n-3 (alpha-linolenic), which uses low irradiance from a dichromatic light source.

## Description

### Field of Invention

The present invention refers to a method to increase lipid production in alga *Chlorella vulgaris* in a controlled way and more specifically it refers to a novel method to increase fatty acids production in alga *Chlorella vulgaris* Beyerinck (Beijerinck) that allows to obtain a significant increase in type C:16 fatty acids (palmitic), C18:1n-9 (oleic), C18:2n-6t (linoleidic) and C18:3n-3 (alpha-linolenic), also favoring an increase in biomass production from algae.

### BACKGROUND OF THE INVENTION

Microalgae cultures are one of the alternatives for generating plant biomass with the greatest potential for development since they do not require solid substrates for their implementation. Also, these cultures have the advantage of being so versatile that they can be carried out in the open air or in closed reactors where culture conditions can be better controlled. One of the fundamental aspects to consider when carrying out these cultures is to make biomass production more efficient, that is, the amount in grams of the harvested cells respect to the volume of liquid medium with nutrients used as culture substrate. The biomass of the cells of the microalgae varies depending on the size of the cells and the organic dry weight (ash-free).

In the state of the art, various methods are described to produce microalgae cultures in which the yield in the amount of biomass produced depends on various factors such as cultivation system type, the magnitude of the environmental variables among which is mainly considered the temperature, the quantity, and the chemical form of the nutrients available in the medium, the salinity, the pH, the microalgae species type, and the lighting conditions.

Microalgae cultures are considered one of the most promising sources of biomass for obtaining biofuels, because many species have rapid growth and high biomass production, as well as a high content of lipids that can be used for production of biodiesel. On the other hand, microalgae use dissolved carbon dioxide and can grow in wastewater using the nutrients available in the same, in addition they can be grown on land unsuitable for agriculture [Christi, Y. (2007) Biodiesel from microalgae. Biotechnology Advances 25(3) :294-306] .

One of the most significant factors in altering the microalgae cell metabolism is the type and rate of light exposure, which refers to the hours of light and darkness (light and dark cycle), the amount of light (irradiance) and the light spectral composition (wavelengths or colors of light). The light spectral composition in some species of microalgae modifies growth, enzyme production, photosynthesis, and metabolic synthesis pathways [Voskresenskaya, N.P. (1972) Blue light and carbon metabolism. Annual Review of Plant Physiology 23(1):219-234.; de la Peña M.R. (2007) Cell growth and nutritive value of the tropical benthic diatom, Amphora sp., at varying levels of nutrients and light intensity, and different culture locations. Journal of Applied Phycology 19:647-655; Vadiveloo, A., N.R. Moheimani, J.J. Cosgrove, P.A. Bahri, D. Parlevliet. (2015) Effect of different light spectra on the growth and productivity of acclimated Nannochloropsis sp. (Eustigmatophyceae). Algal Research 8:121-127]. Additionally, during a microalgae culture, the availability of nutrients and light tends to decrease, because the nutrients are used for the biomass production from the cells and the light decreases due to the increase in density of cells in suspension. Therefore, in microalgae cultures during the exponential growth phase (when there is no limitation of light and nutrients) growth and production biomass are greater than in the growth stationary phase (where there is limitation of light and/or nutrients). These changes in microalgae growth also produce modifications in the cell chemical composition. However, not all microalgae species respond in the same way to different environmental variables and these differences in their response are due to their physiological characteristics, which determine their growth rate, cell size and biochemical composition. These differences in the physiology of the microalgae are due to a species-specific response, since, depending on the light spectrum incident on the crop, different amounts and types of pigments are produced that allow the microalgae to capture the light energy, of which in turn depends on the enzymatic activity and therefore the production of cellular metabolites.

Different studies have been carried out to try to identify how the incident light spectrum on microalgae cultures modifies cell metabolism. For example, Kim et al. 2014 [Kim, D.G., Lee, C., Park, S.M. y Choi, Y.E. (2014) Manipulation of light wavelength at appropriate growth stage to enhance biomass productivity and fatty acid methyl ester yield using Chlorella vulgaris. Bioresource Technology 159:240-248] describe a study on the effect of various types of red (630-665 nm) and blue (430-465 nm) LEDs (Light-emitting diodes) lamps on the phototrophic culture of *Chlorella vulgaris* obtained from the UTEX collection in Texas, USA. The culture was carried out in JM medium in 1000 ml Erlenmeyer at 26±0.5°C, 100 µmol photon m-2 s-1 of continuous light and aeration of 110 ml min-1. The results showed that blue light increased cell size and that red light decreased cell size but activated cell division. The authors propose the alternate use of culture illumination with blue light and red light to promote growth and lipid production compared to the control treatment (white light). In blue light they obtain cells with a size between 4.5 and 6.0 µm, in red light between 1.8 and 4.0 µm and in white light between 2.8 and 5.0 µm. The biomass production as dry weight was 0.020 ng cell-1 for blue light and 0.15 to 0.22 ng cell-1 for red light. In this study, the values are described only for some components of the fatty acid profile and their effect by changing the lighting type at different number of days in blue and red light in which they obtain for C10:0 (0.51 to 1.51%), C14:0 (1.11 to 1.63%), C16:0 (14.38 to 15.53%), C17:1 (11.23 to 12.28%), C18:0 (3.7 a 7.21%), C18:2 (7.76 to 10.33%) and C18:3 (29.7 to 33.47%).

Blair et al. 2014 [Blair, M.F., Kokabian, B. y Gude, V.G., (2014) Light and growth medium effect on Chlorella vulgaris biomass production. Journal of Environmental Chemical Engineering 2(1) : 665-674] describe a study about the effect of different wavelengths of incident light (blue, white, green, red) and the culture medium composition on the microalgae growth. In this study they used a strain of *Chlorella vulgaris* obtained from Connecticut Valley Biological Supply Co. Inc., Southampton, MA. The culture medium used was basal Bold (BBM) and the cultures were maintained in 6 L reactors at 276 µmol photon m-2 s-1, with constant agitation of 100 rpm at controlled temperature of 25°C. The experiments were carried out in 3 phases: 1) the different wavelengths effect 2) the medium effect at 25%, 50% and 100% of the suggested composition and 3) the nutrient concentration effect (nitrogen and phosphorus). The results showed that blue light produces higher growth rates and biomass production compared to that obtained in white, red, and green light. It was found that because of the culture medium, the concentration of 50% respect to the suggested composition is the one that provides the greatest growth. The biomass production values on day 2 resulted for white light in 0.02 g L-1 day-1, for red light 0.005 g L-1 day-1, for blue light 0.012 g L-1 day-1 and for green light 0.01 g L-1 day-1.

Daliry et al. 2017 [Daliry, S., Hallajisani, A., Roshandeh, J.M., Nouri, H., Golzary, A. (2017) Investigation of optimal condition for Chlorella vulgaris microalgae growth. Global Journal of Environmental Science and Management 3(2):217-230], is a bibliographic review work where the behavior of the *Chlorella vulgaris* culture is described when it is maintained in different environmental conditions. It is mentioned that the cultures are more efficient when the source of nitrates is in a concentration between 20 to 0.5 g L-1, pH is set between 9 to 10, lighting is discontinuous between 5 and 7 Klux and, establishes an aeration flow of 200 ml min-1. The highest biomass production obtained was 3.43 g L-1 and the best lipid production was 66.25 mg L-1 day-1. Taken from the work of Kim et al. (2014), describe the changes in the size of *Chlorella vulgaris* cells when exposed to blue light, which increases the diameter between 60 and 70% compared to red light and that biomass production changes depending on the days that are exposure to red or blue light.

Pérez-Pasos et al. 2011 [Pérez-Pazos, J.V., Fernandez-Izquierdo, P. (2011) Synthesis of neutral lipids in Chlorella sp. under different light and carbonate conditions. CT&F-Ciencia, Tecnología y Futuro 4(4):47-58] describe the use of a species of *Chlorella* sp. which is a wild microalga cataloged as LAUN0016 and which was grown in Bold's Basal medium (BBM), maintained in photobioreactors with white light at 20°C, constant aeration and a 12/12 hour period of light and dark. In the work different experimental conditions are used in a factorial design where they use red light (700 nm) and blue (500 nm), 2 concentrations of CaCO3 (0.5 and 1.5 g L-1) and 2 photoperiods (18:6 and 6:18) hours of light and darkness. The results show that the highest growth rate was obtained with red light, with 0.5 g L-1 CaCO3 and 6:18 photoperiod (0.1090±0.0014), while the highest production of lipids occurs with blue light 1.5 g L-1 CaCO3 and 18: 6 photoperiods (0.7783±0.0219 g L-1). In the Article they show a chromatogram, but they do not calculate the lipid percentage for each type of fatty acid in the different experimental conditions. The authors describe the effects of using blue and red light, but only indicate the peak wavelength for each case; however, they do not indicate the type of lamp neither the spectral characteristics of the light used for the tests.

Sanchez-Saavedra et al. 1996 [Sanchez-Saavedra, M.P., Jiménez, C., Figueroa, F.L. (1996) Far-red light inhibits growth but promotes carotenoid accumulation in the green microalgae Dunaliella bardawil. Physiology Plantarum 98:419-423] describe a study with Dunaliella bardawil, where the cultures were exposed to an irradiance gradient of 50 to 300 µmol photon m-2 s-1 of white light and far-red light, it was found that for both types of illumination the cell density and the growth rate increased in direct relation to the increase in irradiance. The growth rate was significantly lower with far red light (in the irradiance gradient) compared to white light however, it was found that carotenoid production increased up to 50% (based on dry weight) and that an increase in cell size was obtained by the effect of far-red light respect to the lower values obtained with white light.

Finally, Chávez-Fuentes et al. (2018) [Chávez-Fuentes, P., Ruiz-Marin, A., Y. Canedo-López. 2018. Biodiesel synthesis from Chlorella vulgaris under effect of nitrogen limitation, intensity, and quality light: estimation on the based fatty acids profiles. Molecular Biology Reports https://doi.org/10.1007/s11033-018-4266-9] discloses that in tests carried out with *Chlorella vulgaris,* exposed to a limiting nitrogen effect and the light spectral composition respect to the composition of fatty acids, a higher biomass production and cell density were found in light irradiance at 140 µE m-2 s-1, in white light and blue light compared to violet and yellow light. In said document it is also mentioned that due to the light spectral composition, no negative effect was found in the production of lipids for use as biofuels. In the fatty acid composition, the main component was C12:0 (lauric acid) in values between 53.09 for blue light in irradiance of 140 µE m-2 s-1 and of 80.33 and 80.86 in white and violet light maintained with 70 µE m-2 s-1, respectively. These fatty acid values indicated for *Chlorella vulgaris* by Chávez-Fuentes et al. (2018) are quite different from those published in the literature of the art even under standard production conditions of in white light.

Although in the above-mentioned Articles, it is described that the type of incident light can modify the metabolic response of microalgae and particularly is indicated it is one of the critical parameters in the production of metabolites, none of them allows a clear overview about the effects of incident radiation on the production of specific compounds. Likewise, in these Articles, the studies focus on specific microalgae species and the irradiance used is high, so the crops obtained would have difficulties to be scaled at an industrial level, since their requirements are very excessive. Furthermore, although the algae studied turn out to be the most promising for obtaining large amounts of biomass, the lipid production profile obtained in the above-mentioned documents is not the most suitable for the biomass obtained to be used in biodiesel production processes.

In view of the above problems, there is a need to provide a specific methodology capable of modifying the metabolic behavior of the alga *Chlorella vulgaris* Beyerinck, which is one of the most suitable microalgae to be used in the biomass production for biofuel obtaining processes, which favors the production of fatty acids with a high cetane (CN) index. Likewise, there is a need to provide a culture methodology in which the incident light power requirements are significantly reduced to favor the large-scale method implementation, while the type of lipids produced is controlled to obtain a biomass with a controlled and reproducible lipid compound profile.

### SUMMARY OF THE INVENTION

To overcome the disadvantages of conventional microalgae cultivation methods, the present invention aims to provide a new specific cultivation methodology that allows increasing the fatty acids production in the algae *Chlorella vulgaris* Beyerinck, without the need to modify the culture medium basal conditions.

A particular object of the present invention is to provide a specific cultivation method for the alga *Chlorella vulgaris* Beyerinck that allows to obtain a significant increase in lipid production of type C:16 (palmitic), C18:1n-9 (oleic), C18:2n-6t (linoleidic) and C18:3n-3 (alpha-linolenic).

Another objective of the present invention is to provide a method to increase the fatty acids production in alga *Chlorella vulgaris* Beyerinck, which uses a low irradiance power, and which also allows to increase the produced biomass.

An additional objective of the present invention is to provide a method to increase the fatty acids production in the alga *Chlorella vulgaris* Beyerinck, which allows obtaining a biomass with a high proportion of lipids that can be used in biofuel obtaining processes.

The above mentioned, as well as other, objects and advantages of the present invention will become apparent from the following detailed description thereof.

### DETAILED DESCRIPTION OF THE INVENTION

The present invention provides a novel method to increase the fatty acids production in alga *Chlorella vulgaris* Beijerinck, particularly to increase the lipid production of type C:16 (palmitic), C18:1n-9 (oleic), C18:2n -6t (linoleidic) and C18:3n-3 (alpha-linolenic); while the crop yield is increased, obtaining a higher biomass.

The method of the present invention allows to control the metabolism of the alga *Chlorella vulgaris* Beyerinck, to favor the production of C:16 type fatty acids (palmitic), C18:1n-9 (oleic), C18:2n-6t (linoleidic) and C18: 3n-3 (alpha-linolenic), without it being necessary to modify in any way the conventional algae culture media basal conditions. For this, the method of the present invention uses low intensity dichromatic lighting sources with specific wavelengths applied in specific growth phases, which allow modifying the basal metabolic behavior of the algae in such a way that the production of the above-mentioned fatty acids is favored.

The method of the present invention comprises the following sequential steps:
1.- Add an inoculum of the alga *Chlorella vulgaris* Beyerinck in exponential growth phase, to a culture medium "f" [(Guillard, R.R.L., Ryther, J.H. (1962). Studies of marine planktonic diatoms: I. Cyclotella nana Hustedt, and Detonula confervacea (CLEVE) Gran. Canadian Journal of Microbiology, 8 (2):229-239)], prepared with fresh water at pH between 7.3 and 8.0, until obtaining a concentration between 0.4 to 0.5 x 106 cells ml- 1;
2.- Illuminate the solution from step 1 with a dichromatic light source with emission peaks of 450 nm and 625 nm, at irradiance of 50 µmol photon m-2 s-1, continuously in light/dark cycles of 24:0, maintaining the culture at temperature between 20°C and 22°C for a period between 48 and 72 hours until obtaining a cell density between 0.95 to 1.05 X 106 cells ml-1;
3.- Centrifuge the obtained biomass between 3800 and 4200 rpm, for 5 to 10 min at temperature between 4°C and 6°C and;
4.- Lyophilize the centrifuged biomass at -50°C and at pressure of 0.11 bar to obtain dry cells of *Chlorella vulgaris* Beyerinck with a high percentage of fatty acids.

With the above-described method, a dry biomass is obtained that has complete cells of *Chlorella vulgaris* Beyerinck with an average biomass production of 57.2 pg cell-1. This biomass obtained has a total lipid content of 18% in the exponential phase and 11% in the stationary phase, with the lipid content of C:16 being 12.47% in the exponential phase and 19.71% in the stationary phase, C18: 1n-9 of 12.09% in the exponential phase and 23.23% in the stationary phase, C18: 2n-6t of 12.04% in the exponential phase and 10.42% in the stationary phase and C18: 3n-3 of 36.31% in the exponential phase and 25.89 in the stationary phase. Said lipid content, as well as its type, allow the biomass obtained to be used as raw material for conventional biofuel obtaining processes, given that the type of lipids obtained present the best profile to be used as starting material of processes for obtaining high cetane index oils. Likewise, since no type of growth accelerating hormones are used or toxic compounds are included in the culture medium, the biomass obtained can be used in the manufacture of animal feed or supplements intended for human consumption. In addition to the above, by not using strains with genetic modifications of *Chlorella vulgaris* Beyerinck, the environmental safety and innocuousness of the obtained biomass is guaranteed.

The biomass obtained by the methodology described above is perfectly susceptible to be used in biodiesel obtaining processes since fatty acids with a high degree of saturation (SFAs) are obtained as in the case of C16: 0 with a percentage concentration in exponential phase of 12.47 ± 0.15 and in the stationary phase of 19.71 ± 0.73, which show high value of cetane number (CN = 74.5), high values of melting point (62.9°C), high boiling point (351.0°C) and low density (0.85 g cm-3). The number of cetanes (CN) is one of the indicators that describe the combustion biodiesel quality during the ignition process. Particularly in biodiesel engineering, a high CN value in fuels has short ignition retardation processes compared to low CN values (Knothe, 2005). [Knothe, G. (2005) Dependence of biodiesel fuel properties on the structure of fatty acid alkyl esters. Fuel processing Technology. 86(10):1059-1070]. The CN and the ignition quality are inversely related by means of a negative logarithmic function respect to the fatty acid saturation degree (Knothe et al., 2003) [Knothe. G., Matheaus, A.C., Ryan T.W. (2003) Cetane numbers of branched and straight-chain fatty esters determined in an ignition quality tester. Fuel 82(8):971-975]. The saturated fatty acids (SFAs) produced with the method above described, have high CN values, and stabilize combustion with low emissions, are resistant to degradation and consequently increase the longevity of the biodiesel obtained, they also increase oxidation resistance in places with hot climates, so the biomass obtained by the methodology above described can be used in conventional biodiesel obtaining processes.

The biomass obtained by the method of the present invention can also be used as a food supplement, since the fatty acids type and proportion obtained make it an ideal candidate as a starting raw material for obtaining feed and supplements. Trough the methodology of the present invention, a saturated fatty acid rich biomass is obtained that are stable to oxidation because of the saturation of the carbon double bonds, so this biomass can be used to provide important characteristics in the food stability. For example, palmitic fatty acid is used in human diet and constitutes about 60% of their diet, its contribution is mainly meat and animal fats (butter, cheese, cream) as well as vegetables such as palm (44%) and seeds (8 to 20%) [Carta, G., E. Murru, S. Banni, C. Manca. 2017. Palmitic acid: physiological role metabolism and nutritional implications. Frontiers in Physiology 8, 902 https://doi.org/10.3389/fphys.2017.00902]. On the other hand, oleic fatty acid is used as a hypotensive and prevents the development of cardiovascular problems and helps control cholesterol and high-density lipoproteins (HDL) [Pérez-Rosales, R., Villanueva-Rodriguez S., Cosio-Ramirez, R. 2005. Avocado oil and its nutritional properties. E-Gnosis 3(10): 11 pp.].

To demonstrate the technical advantages of the above-mentioned method, an example of the implementation of the present invention is shown below.

Example 1. Cultivation of the alga *Chlorella vulgaris* Beyerinck under the present invention methodology.

A monospecific mother crop of *Chlorella vulgaris* Beyerinck (Beijerinick) 1890 cells obtained from the microalgae collection of the "Institute of Applied Microbiology (IAM)" of Tokyo University, Bunkyo-Ku, Tokyo, Japan was started.

Cells were taken from the mother crops in exponential growth phase and transferred to Erlenmeyer flasks containing culture medium "f" (Guillard and Ryther, 1962), prepared with fresh water, the composition of which is shown in Table 1.

**Table 1. Culture medium composition "f".**

| Compound | Concentration |
|---|---|
| NaNO3 | 150 mg |
| NaH2PO4•H2O | 10 mg |
| Fe Chelate | 10 mg |
| Na2SiO3•9H2O | 30-60 mg |
| Thiamine•HCl | 0.2 mg |
| Biotin | 1.0 mg |
| B12 | 1.0 mg |
| CuSO4•5H2O | 0.0196 mg |
| ZnSO4•7H2O | 0.044 mg |
| CoCl2•6H2O | 0.020 mg |
| MnCl2•4H2O | 0.360 mg |
| NaMoO4•2H2O | 0.0126 mg |
| Water | cbp 1 L |

The cultures were started with an average cell density between 0.40 to 0.42 x 106 cells ml-1 and were irradiated with a dichromatic light source with emission peaks at 450 nm and 625 nm, for light and dark photoperiods of 24:0 continuously, maintaining the temperature of the cultures at 21±1°C and pH between 7.3 and 8.0, with manual shaking for 1 minute every 24 hours.

Cell density was measured daily by direct counts with a hemocytometer and compound microscope, and cell size was assessed by randomly measuring cell diameter.

The evaluation of biomass amount produced from *Chlorella vulgaris* Beyerinck was carried out by collecting an aliquot of the cultures on glass fiber filters (GF/C) with a pore opening of 1 µm, the filter was rinsed with ammonium formate (3%) to eliminate salt residues. The total dry weight (TDW) was obtained by keeping the filters in an oven at 60°C until constant weight was obtained. The ash content (AC) was obtained by incinerating the filters in a muffle at 450°C for 12 h to subsequently obtain the sample weight. The content of the organic dry weight (ODW) was calculated by subtracting the value of the total dry weight from the ash weight ODW = TDW-AC).

The biomass production per cell was calculated by relating the sample organic weight and the cell density. The daily biomass production (DBP) was obtained by relating the organic dry weight with the cultivation time.

The lipid content of the *Chlorella vulgaris* Beyerinck cultures was obtained by collecting an aliquot of a volume of the cultures on glass fiber filters (GF/C) with a pore opening of 1 µm. The lipids were extracted by macerating the sample and using chloroform, methanol, and water (2:2:1). The quantification was carried out following the sulfuric acid method. The lipid content was expressed as a percentage based on the organic dry weight of the sample. Tripalmitin (99%) was used as standard for the calibration curve. To obtain the fatty acid profile, the biomass of *Chlorella vulgaris* Beyerinck cells was harvested by centrifugation at 4000 rpm, for 5 to 10 min and at 4°C. The wet biomass of the *Chlorella vulgaris* Beyerinck cells was lyophilized at -50°C and pressure of 0.110 bar until obtaining dry biomass. The extraction of lipids from *Chlorella vulgaris* Beyerinck cells was carried out by cold gravimetric methods using chloroform for extraction. Methylation was performed and the fatty acid profile was analyzed by gas chromatography. For identification of the fatty acids, the fatty acids retention time of a commercial standard was taken into consideration.

All the titrations were carried out in the exponential growth phase (E) day 2 and stationary (S) day 8. The results of the culture titrations are shown below:

**Table 2. Growth rate (µ: divisions day-1), maximum cell concentration (MCC: x 106 cells ml-1), total dry weight (TDW: pg cell-1), organic dry weight (ODW: pg cell -1), cell size (CS: µm) and daily biomass production values (DBP: g L-1 day-1) for Chlorella vulgaris Beyerinck in growth phase: exponential (E) and stationary (S).**

| µ | MCC | TDW | | ODW | | CS | | DBP | |
|---|---|---|---|---|---|---|---|---|---|
| | | E | S | E | S | E | S | E | S |
| 0.47± 0.04b | 2.29± 0.15 | 80.90± 3.37 | 68.51± 5.99 | 57.20± 0.82 | 68.51± 5.99 | 14.95± 2.94 | 14.86± 2.40 | 0.065± 0.001 | 0.078± 0.001 |

**Table 3. Lipid content (%) of Chlorella vulgaris Beyerinck in two growth phases: exponential (E) and stationary (S).**

| Lipids | |
|---|---|
| E | S |
| 18.35±0.25 | 11.27±0.70 |

**Table 4. Fatty acid composition (based on the percentage of total fatty acids) of Chlorella vulgaris Beyerinck grown in two growth phases: exponential and stationary.**

| Lipid type | Growth phase | | | |
|---|---|---|---|---|
| Saturated Fatty Acids (SFA) | Exponential | | Stationary | |
| C6:0 | 0.13 | ±0.01 | 0.15 | ±0.01 |
| C8:0 | 0.49 | ±0.03 | 0.36 | ±0.02 |
| C10:0 | 3.61 | ±0.10 | 3.02 | ±0.12 |
| C11:0 | 0.96 | ±0.03 | 1.06 | ±0.03 |
| C12:0 | 0.13 | ±0.01 | | |
| C13:0 | 1.89 | ±0.08 | 1.56 | ±0.05 |
| C14:0 | 0.38 | ±0.01 | 0.38 | ±0.01 |
| C15:0 | 0.18 | ±0.00 | 0.19 | ±0.01 |
| C16:0 | 12.47 | ±0.15 | 19.71 | ±0.73 |
| C17:0 | 0.19 | ±0.01 | 0.23 | ±0.02 |
| C18:0 | 1.74 | ±0.10 | 2.09 | ±0.03 |
| C20:0 | 0.21 | ±0.01 | 0.30 | ±0.03 |
| C21:0 | | | | |
| C24:0 | 0.11 | ±0.01 | 0.07 | ±0.00 |

| Monounsaturated fatty acids | | | | |
|---|---|---|---|---|
| C14:1 | 0.43 | ±0.02 | 0.33 | ±0.01 |
| C15:1 | 0.33 | ±0.02 | 0.08 | ±0.01 |
| C16:1 | 9.25 | ±0.48 | 8.13 | ±0.05 |
| C17:1 | 6.18 | ±0.22 | 1.56 | ±0.09 |
| C24:1 | 0.06 | ±0.00 | 0.09 | ±0.00 |
| C18:1n-9 | 12.09 | ±0.13 | 23.23 | ±0.41 |
| C20:1n-9 | 0.11 | ±0.01 | 0.30 | ±0.00 |
| C22:1n-9 | 0.18 | ±0.01 | 0.38 | ±0.01 |

| Polyunsaturated fatty acids (PUFA) | | | | |
|---|---|---|---|---|
| C18:2n-6c | 0.19 | ±0.01 | 0.09 | ±0.00 |
| C18:2n-6t | 12.04 | ±0.11 | 10.42 | ±0.09 |
| C18:3n-6 | 0.19 | ±0.00 | 0.10 | ±0.01 |
| C18:3n-3 | 36.31 | ±0.73 | 25.89 | ±0.81 |
| C20:2 | | | 0.08 | ±0.01 |
| C20:3n-3 | 0.08 | ±0.00 | 0.12 | ±0.00 |
| C20:4n-6 | | | | |
| C20:5n-3 | 0.06 | ±0.00 | 0.08 | ±0.00 |
| TOTAL | 100.00 | | 100.00 | |

The previous results unequivocally show that, with the methodology of the present invention, the proportion of fatty acids produced by *Chlorella vulgaris* Beyerinck can be increased, obtaining a lipid profile suitable for use as a starting material in biodiesel obtaining processes and with high cetane index values.

The present invention has been described in accordance with one of its preferred embodiments; however, it will be apparent to a person skilled in the art that modifications can be made to the invention without departing from its spirit and scope.

## Claims

1. A method to increase the fatty acids production in *Chlorella vulgaris* Beyerinck and obtain a biomass from the algae with a high lipid percentage of C: 16, C18: 1n-9, C18: 2n-6t and C18: 3n-3 **characterized in that** it comprises the steps of:
1) Add an inoculum of the alga *Chlorella vulgaris* Beyerinck in exponential growth phase, to a culture medium "f", prepared with fresh water at pH between 7.3 and 8.0, until obtaining a concentration between 0.4 to 0.5 x 106 cells ml -1;
2) Illuminate the solution from step 1 with a dichromatic light source with emission peaks of 450 nm and 625 nm, at irradiance of 50 µmol photon m-2 s-1, continuously in light/dark cycles of 24:0, maintaining the culture at temperature between 20°C and 22°C for a period between 48 and 72 hours until obtaining a cell density between 0.95 to 1.05 X 106 cells ml-1;
3) centrifuge the obtained biomass at between 3800 and 4200 rpm, for 5 to 10 min at temperature between 4°C and 6°C and;
4) lyophilize the centrifuged biomass at -50°C and at pressure of 0.11 bar to obtain *Chlorella vulgaris* Beyerinck cells with a total lipid content of 18%, C20 lipids 31%, C16 lipids 17% and C18: 1n9t lipid 20.63%.

2. The method according to claim 1, **characterized in that** the culture medium "f" comprises 150 mg of NaNO3; 10 mg of NaH2PO4•H2O; 10 mg of Fe chelate; between 30 to 60 mg of Na2SiO3•9H2O; 0.2 mg thiamine•HCl; 1.0 mg of biotin; 1.0 mg of B12; 0.0196 mg of CuSO4•5H2O; 0.044 mg of ZnSO4•7H2O; 0.020 mg of CoCl2•6H2O; 0.360 mg of MnCl2•4H2O; 0.0126 mg of NaMoO4•2H2O and; 1L cbp water.

3. The method according to claim 1, **characterized in that** the culture of step 2) is maintained at temperature of 21°C and the pH between 7.3 and 8.0, with manual stirring for 1 minute every 24 hours.

4. The method according to claim 1, **characterized in that** the centrifugation of step 3 is carried out at 4000 rpm and temperature of 4°C.

5. The method according to claim 1, **characterized in that** the lyophilized *Chlorella vulgaris* Beyerinck cells have a lipid profile based on the total percentage of fatty acids in the exponential phase of 0.13 for C6:0; 0.49 for C8:0; 3.61 for C10:0; 0.96 for C:11; 0.13 for C12:0; 1.89 for C13:0; 0.38 for C14:0; 0.18 for C:15; 12.47 for C16:0; 0.19 for C17:0; 1.74 for C18:0; 0.21 for C:20; 0.11 for C:24; 0.43 for C14:1; 0.33 for C15:1; 9.25 for C16:1; 6.18 for C17.1; 0.06 for C24:1; 12.09 for C18:1n-9; 0.11 for C20:1n-9; 0.18 for C22:1n-9; 0.19 for C18:2n-6c; 12.04 for C18:2n-6t; 0.19 for C18:3n-6; 36.31 for C18:3n-3; 0.00 for C20:2; 0.08 for C20:3n-3; 0.06 for C20:5n-5. The lipid profile during the stationary phase corresponds to 0.15 for C6:0; from 0.36 for C8:0; 3.02 for C10:0; 1.06 for C:11; 0.00 for C12:0; 1.56 for C13:0; 0.38 for C14:0; 0.19 for C:15; 19.71 for C16:0; 0.23 for C17:0; 2.09 for C18:0; 0.30 for C:20; 0.07 for C:24; 0.33 for C14:1; 0.08 for C15:1; 8.13 for C16:1; 1.56 for C17.1; 0.09 for C24:1; 23.23 for C18:1n-9; 0.30 for C20:1n-9; 0.38 for C22:1n-9; 0.09 for C18:2n-6c; 10.42 for C18:2n-6t; 0.10 for C18:3n-6; 25.89 for C18:3n-3; 0.08 for C20:2; 0.12 for C20:3n-3; 0.08 for C20:5n-5.

6. The use of *Chlorella vulgaris* Beyerinck cells obtained from step 4, to produce biofuels.

7. The use of *Chlorella vulgaris* Beyerinck cells obtained from step 4, to prepare a food supplement.
